# EUROPÄISCHE PATENTSCHRIFT

(11) **EP 0 826 695 B1**
(45) Veröffentlichungstag und Bekanntmachung des Hinweises auf die Patenterteilung: **12.12.2001**
(21) Anmeldenummer: 97115188.1
(22) Anmeldetag: 02.09.1997
(51) Int. Cl.: C07K 16/46, A61K 39/395

(54) **Zerstörung von kontaminierenden Tumorzellen in Stammzelltransplantaten mit bispezifischen Antikörpern**
Destruction of contaminating tumor cells in stem cell transplants by use of bispecific antibodies
Destruction de cellules tumorales contaminantes dans un transplant de cellules souches avec des anticorps bispecifiques

(30) Priorität: 03.09.1996 DE 19635743; 27.11.1996 DE 19649223
(43) Veröffentlichungstag der Anmeldung: 04.03.1998
(73) Patentinhaber: GSF-Forschungszentrum für Umwelt und Gesundheit GmbH, 85764 Oberschleissheim (DE)
(72) Erfinder: Lindhofer, Horst, Dr., 82194 Gröbenzell (DE); Menzel, Helge, Dr., 81379 München (DE); Kolb, Hans-Jochem, Prof. Dr., 80804 München (DE); Thierfelder, Stefan, Prof. Dr., 82223 Eichenau (DE)
(74) Vertreter: Reinhard - Skuhra - Weise & Partner

(56) Entgegenhaltungen:
- EP-A- 0 637 593
- LINDHOFER H. ET AL.: "Bispecific Antibodies Target Operationally Tumor-Specific Antigens in Two Leukemia Relapse Models" BLOOD, Bd. 88, 15.Dezember 1996, Seiten 4651-4658, XP000616201
- LINDHOFER,H. ET AL: "Bispecific antibodies effectively purge cancer cells from peripheral blood stem cell collections without affecting colony forming units " 26TH ANNUAL MEETING OF THE INTERNATIONAL SOCIETY FOR EXPERIMENTAL HEMATOLOGY, CANNES, FRANCE, AUGUST 24-28, 1997, Bd. 25, Nr. 8, 1997, EXPERIMENTAL HEMATOLOGY (CHARLOTTESVILLE), Seite 879 XP002048523
- KANEKO T. ET AL.: "Combination of interleukin-2-stimulated lymphocytes and bispecific antibodies that efficiently lyse leukemic cells does not affect bone marrow CD34-positive stem cell function in vitro" BONE MARROW TRANSPLANTATION, Bd. 14, 1994, Seiten 213-217, XP002048524
- KANEKO T. ET AL.: "Cytotoxicity of cytokine-induced killer cells coated with bispecific antibody against acute myeloid leukemia cells" LEUKEMIA AND LYMPHOMA, Bd. 14, 1994, Seiten 219-229, XP002048525
- KANEKO T. ET AL.: "A bispecific antibody enhances cytokine-induced killer-mediated cytolysis of autologous acute myeloid leukemia cells" BLOOD, Bd. 81, 1993, Seiten 1333-1341, XP002048526
- WEINER G.J. ET AL.: "The role of Tcell activation in anti-CD3 X antitumor bispecific antibody therapy" JOURNAL OF IMMUNOLOGY, Bd. 152, 1994, Seiten 2385-2392, XP002048527
- WEINER, G.J. AND DE GAST G.C.: "Bispecific monoclonal antibody therapy of B-cell malignancy" LEUKEMIA AND LYMPHOMA, Bd. 16, 1995, Seiten 199-207, XP002048528
- WEINER L.M. ET AL.: "Clinical development of 2B1, a bispecific murine monoclonal antibody targeting c-erbB-2 and Fc-gamma-RIII" JOURNAL OF HEMATOTHERAPY, Bd. 4, 1995, Seiten 453-456, XP002048529
- SILLA L.M.R. ET AL.: "Potentiation of lysis of leukaemia cells by a bispecific antibody to CD33 and CD16 (Fc-gamma-RIII) expressed by human natural killer (NK) cells" BRITISH JOURNAL OF HAEMATOLOGY, Bd. 89, 1995, Seiten 712-718, XP002048530
- CHEN J. ET AL: "MONOCYTE-MEDIATED LYSIS OF ACUTE MYELOID LEUKEMIA CELLS IN THE PRESENCE OF THE BISPECIFIC ANTIBODY 251 X 22 (ANTI-CD33 X ANTI-CD64 )" CLINICAL CANCER RESEARCH, Bd. 1, Nr. 11, 1995, Seiten 1319-1325, XP000608204

## Beschreibung

Die Erfindung betrifft ein Verfahren zur Zerstörung von kontaminierenden Tumorzellen in Stammzelltransplantaten ex vivo unter Verwendung intakter bispezifischer Antikörper.

Bei etwa 43.000 Neuerkrankungen/Jahr steht Brustkrebs an der Spitze der Krebsstatistik für Frauen in Deutschland. Weniger als ein Drittel der Frauen mit Lymphknotenbefall bei Diagnose leben 10 Jahre ohne Rückfall.

Vor diesem Hintergrund wird seit einigen Jahren versucht, mit Hilfe der autologen Knochenmark- und Blutstammzell-Transplantation in Verbindung mit der Hoch-Dosis-Therapie Patientinnen mit ausgedehntem Lymphknotenbefall und Fernmetastasen eine Lebensverlängerung oder sogar Heilung zu ermöglichen. Trotz hoher Ansprechraten bei der Hoch-Dosis-Therapie ist eine Heilung im metastasierten Stadium selten.

Die Therapie des metastasierten Mammakarzinoms ist heute fast ausschließlich palliativ. In klinischen Phase-1-Studien konnte gezeigt werden, daß eine Hochdosis-Chemotherapie (HD-CT), gefolgt von einer autologen Knochenmark- oder Stammzelltransplantation (aPBSZT), bei Patientinnen mit chemotherapie-sensitivem metastasierten Mammakarzinom klinische Vollremissionen herbeiführen kann. Die Remissionen sind jedoch zeitlich begrenzt, meistens kommt es zu einem Rezidiv. Dabei kann ein Rezidiv aus klonogenen Tumorzellen entstammen, die mit dem Transplantat reinfundiert wurden und/oder aus solchen, die die HD-CT in der Patientin überlebt haben. Mikrometastasen können im Knochenmark nach Chemotherapie mit der sensitiven RT-PCR für CK19 bzw. ep-cam, beispielsweise C215, und mit Immunzytologie nachgewiesen werden. Sie sind mit einer ungünstigen Prognose selbst nach HD-CT und autologer Stammzelltransplantation verknüpft. Neue Konzepte zur Elimination von minimal residual disease (MRD) und zur Reinigung der Transplantate von kontaminierenden Tumorzellen erscheinen daher notwendig. Nach Erreichen einer Remission besteht MRD vermutlich überwiegend aus Tumorzellen, die gegenüber antiproliferativer Chemotherapie durch Verharren in einem Ruhezustand (kinetische Resistenz) oder Ausprägung biochemischer Mechanismen, wie z.B. multi drug resistance (MDR), resistent sind.

Ein wesentliches Problem bei der autologen Stammzelltransplantation ist die Kontamination des Transplantats mit Tumorzellen, welche zum Auftreten eines späteren Rezidivs beim Patienten beitragen können (7,8). Um Stammzelltransplantate von kontaminierenden Tumorzellen zu reinigen, wurde bisher hauptsächlich das "Purging" mit immunomagnetischen Beads eingesetzt. Dabei werden Tumorzellen aufgrund gebundener, eisentragender Antikörper an einem Magneten zurückgehalten und damit aus dem Transplantat entfernt. Nachteile sind der hohe zeitliche und technische Aufwand und die damit verbundenen hohen Kosten (ca. 20.000.- DM/Patient). Nach einer solchen in vitro-Verminderung der Zahl residueller Tumorzellen kann dennoch - wenn auch verzögert - ein Rezidiv auftreten. Dies liegt wahrscheinlich an der Beschränkung dieser Methode auf das Stammzelltransplantat sowie an Tumorzellen, die sich einem derartigen mechanistischen Ansatz, z.B. durch "Verklumpung mit normalen Zellen", entziehen.

Aus diesen Gründen wurden andere immunologische Ansätze zur Reinigung des Stammzelltransplantats erprobt, wie z.B. die Zugabe aktivierter T-Zellen in Kombination mit bispezifischen F(ab')2-Fragmenten zur Redirektion von T-Zellen an Tumorzellen (6) in vitro. Es zeigte sich, daß hämatopoetische Stammzellen durch ein derartiges Purgen zwar nicht in ihrer Funktion - gemessen in Proliferations-Assays - beeinträchtigt werden. Die Fähigkeit zur Tumorzell-Zerstörung war in diesen Versuchen jedoch relativ beschränkt (1-2 log Tumorreduktion). Als Nachteil dieses Ansatzes muß auch die Verwendung von zwei Wochen lang kultivierten, präaktivierten T-Zellen angesehen werden, welche den Aufwand hochtreiben und eine Anwendung in der Klinik erschweren.

Es ist eine Aufgabe der vorliegenden Erfindung, ein neues Verfahren zur Verminderung der Anzahl kontaminierender Tumorzellen in Stammzellpräparaten bereitzustellen, das die aus dem Stand der Technik bekannten Nachteile vermeidet.

Diese Aufgabe wird erfindungsgemäß durch das im Anspruch 1 gekennzeichnete Verfahren gelöst. Bevorzugte Ausgestaltungen des Verfahrens ergeben sich aus den Unteransprüchen und aus der nachfolgenden Beschreibung mit den Beispielen.

Die anliegende Abbildung dient zur weiteren Veranschaulichung der Erfindung.

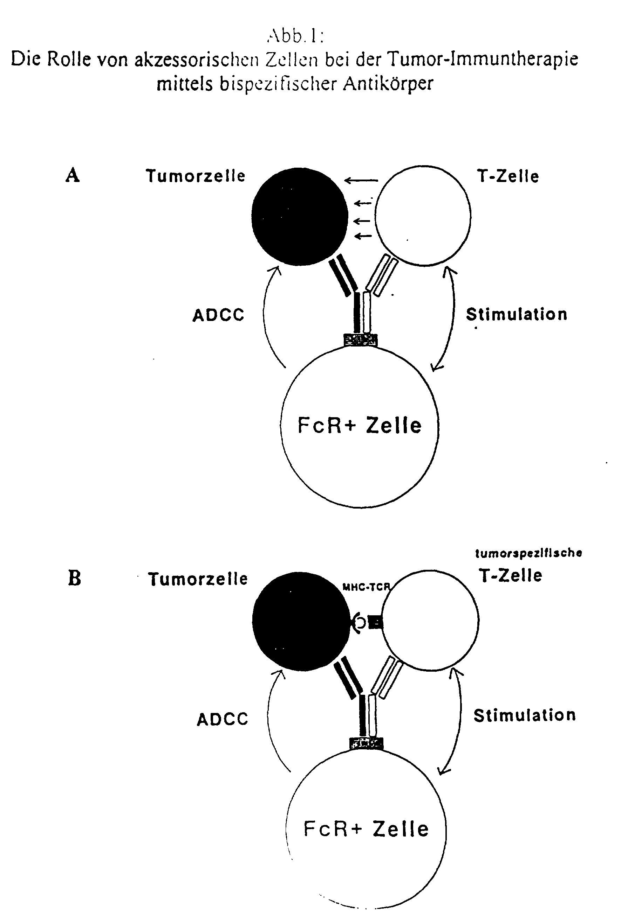

Die Abbildung 1 zeigt die Rolle von akzessorischen Zellen bei der Tumor-Immuntherapie mittels bispezifischer Antikörper (ADCC = Antibody dependent cell mediated Cytotoxicity).

Bispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen die Tumorzellen zerstören. Darüber hinaus besteht die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird. Von besonderer Bedeutung ist dabei der intakte Fc-Teil des bispezifischen Antikörpers, der die Bindung an akzessorische Zellen wie z.B. Monozyten/Makrophagen/Dendriten vermittelt und diese veranlaßt, selbst zytotoxisch zu werden und/oder gleichzeitig wichtige kostimulatorische Signale an die T-Zelle weiterzugeben (siehe Abb. 1).

Erfindungsgemäß werden im Gegensatz zum Stand der Technik intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Im erfindungsgemäßen Verfahren werden kontaminierende Tumorzellen in Stammzellpräparaten (Leukaphereseprodukten) mittels bispezifischer Antikörper (wie z.B. anti-CD3 X anti-c-erbB-2, anti-CD3 X anti-Lewis Y und anti-CD3 X anti-ep-cam, beispielsweise anti-CD3 X anti-C215) in vitro eliminiert. Das Inkontaktbringen der bispezifischen Antikörper und der Stammzellen mit den kontaminierenden Tumorzellen erfolgt unter solchen Bedingungen, die sowohl eine Bindung der bispezifischen Antikörper an die Tumorzellen und die T-Zellen als auch eine Beibehaltung der Lebensfähigkeit der Stammzellen gestatten. Die Einhaltung dieser Parameter ist für die Erhaltung und die Vitalität der Stammzellen wie auch der Lymphozyten notwendig. Beispielsweise wird das Stammzelltransplantat (Leukaphereseprodukt) etwa 4 - 72 Stunden, bevorzugt 24 - 48 Stunden, mit bispezifischen Antikörpern bei Raumtemperatur und einer Zelldichte von 30.000 - 75.000 Zellen/µl, bevorzugt 30.000 - 50.000 Zellen/µl, unter leichtem Schwenken inkubiert. Bei einer Gesamtzellzahl von ca. 10 x 10⁹ Zellen/Stammzelltransplantat ist eine bsAk-Menge von 100 - 500 µg für die Tumorzellzerstörung ausreichend. Ein weiterer wichtiger Punkt des erfindungsgemäßen Verfahrens ist die Verwendung von sogenannten intakten bispezifischen Antikörpern. Diese sind nicht nur in der Lage (aufgrund der hier verwendeten Spezifitäten) T-Zellen an die Tumorzellen zu führen, sondern sie sind aufgrund der Effektorfunktionen des Fc-Teils darüber hinaus geeignet, mittels Complement vermittelter Lyse oder durch Bindung von Fc-Rezeptor positiven Zellen, wie z.B. Makrophagen, Monozyten oder aktivierten neutrophilen Granulozyten, Tumorzellen zu zerstören. Es können somit durch intakte bsAk mehrere tumorzell-zerstörende Mechanismen gleichzeitig aktiviert werden.

Die in der vorliegenden Erfindung verwendeten intakten bispezifischen Antikörper tragen einen funktionellen Fc-Teil. Im Gegensatz zu bispezifischen F(ab)2-Fragmenten, die keinen Fc-Teil besitzen und lediglich T-Zellen an die Tumorzelle heranführen können, sind die in der vorliegenden Erfindung verwendeten intakten bispezifischen Antikörper befähigt, nicht nur T-Zellen zu binden, sondern auch akzessorische Zellen, die als Fc-Rezeptor positive Zellen (beispielsweise Monozyten, Makrophagen und dendritische Zellen) bekannt sind. Die Bindung der Zellen spielt eine essentielle Rolle bei der erfindungsgemäß bewirkten direkten Tumorzerstörung, die um den Faktor 10 - 1000 höher ist als bei Verwendung des von Kaneko et al. beschriebenen Verfahrens. Die erfindungsgemäß verwendeten intakten bispezifischen Antikörper ermöglichen eine optimale Kostimulation der herangeführten T-Zellen durch diese akzessorischen Zellen. Für diese optimale Kostimulation verantwortlich sind insbesondere Oberflächenantigene wie CD40, B-7.1, B-7.2 und LFA-3 und bestimmte sekretierte Zytokine wie IL-2, IL-6, IL-12 und TNF-alpha. Durch die erfindungsgemäße Verwendung intakter bispezifischer Antikörper ist eine wirksame direkte Zerstörung der Tumorzellen durch T-Zellen möglich und weiterhin wird eine mögliche Immunantwort gegen den Tumor eingeleitet. Durch die akzessorischen Zellen kommt es zu einer Aufnahme, Prozessierung und Präsentation von Tumorbestandteilen. Diese durch die erfindungsgemäße Verwendung bispezifischer intakter Antikörper initiierten Schritte sind wesentlich für die Induktion einer humoralen und zellulären Immunantwort mit verantwortlich.

Durch die erfindungsgemäße Verwendung intakter bispezifischer Antikörper wird nicht nur ein quantitativer Effekt, sondern auch eine neue Qualität in Richtung Induktion einer Immunantwort gegen den Tumor erreicht.

Durch die erfindungsgemäße Verwendung intakter bispezifischer Antikörper kann eine Vorbehandlung der T-Zellen durch zusätzliche Zytokine wie IL-2 vermieden werden, wodurch eine beispielsweise zweiwöchige Kultivierung dieser Zellen vermieden wird. Da für eine Anwendung bei Patienten die Kultivierung von T-Zellen unter GMP-Bedingungen ablaufen müßte, die einen wesentlichen Kostenfaktor darstellen und von einer Behörde wie dem Paul-Ehrlich Institut oder der FDA zugelassen werden müßte, stellt das erfindungsgemäße Verfahren eine wesentliche Vereinfachung, Verkürzung und Kostenersparnis dar. Zur Zeit steht kein vergleichbares Verfahren zur Verfügung, um eine wirksame Zerstörung von Tumorzellen in Stammzelltransplantaten zu erreichen.

### Beispiel

Um die Wirksamkeit von intakten bsAk bei der Tumorzellzerstörung zu quantifizieren, wurden den Leukaphereseprodukten (10⁸ Zellen) eines normalen Spenders (siehe Tabelle 1, Spender 2) und zweier Leukämiepatienten eine definierte Menge (0,1%) von Tumorzellen (Brustkrebs-Zellinie MCF-7) beigemischt. Nach 48 h Inkubation des jeweiligen Zellgemisches mit 4µg bsAk (bzw. keinem Antikörper oder der äquimolaren Menge der parentalen Ausgangsantikörper) bei Raumtemperatur und einer Zelldichte von 50.000 Zellen/µl unter leichtem Schwenken wurden die Zellen in verschiedenen Zelldichten auf 96er (10⁵ Zellen/Loch) und 24er (3x10⁶ bzw. 10⁶ Zellen/Loch) NUNC®-Zellkultur-Flachbodenplatten ausplattiert. Wie sich nach 2-wöchiger Kultivierung zeigte, war nur der bsAk in der Lage, das Tumorzellwachstum um den Faktor 1.000 - 10.000 (log 3 - 4), gemessen an der Anzahl der Tumorzellkolonien /plattierten Zellen, zu reduzieren. Das Ergebnis des Tumorkolonie-Wachstums-Assays (Colonogenic A) ist in der nachfolgenden Tabelle 1 zusammengefaßt.

**Tabelle1:**

| Tumorkolonie-Wachstums-Assay | | | | |
|---|---|---|---|---|
| Patient 1 | | | | |
| Platte | kein Anti-körper | parentale Antikörper C215, anti-CD3 | bispezifischer Antikörper BiUII anti-CD3XC215 | Tumorzellen/ MNCs/Loch (= 0.1%) |
| 24 | 6/6^{a} | n.d. | 0/6 Σ=1.8x10⁷ | 3000/3 x 10e6 |
| 24 | 6/6 | n.d. | 0/6 | 1000/10e6 |
| 96 | 12/12 | n.d. | 0/12 | 500/5 x 10e5 |
| 96 | 10/12 | n.d. | 0/12 | 100/10e5 |
| **Tumor reduktion :** | **-** | | **>4 log** | |
| | | | | |

| Spender 2 | | | | |
|---|---|---|---|---|
| 24 | 6/6 | | 0/6 Σ=3x10⁷ | 5000/5 x 10e6 |
| 24 | 6/6 | n.d. | 0/6 | 1000/10e6 |
| 96 | 12/12 | n.d. | 0/12 | 500 / 5 x 10e5 |
| 96 | 12/12 | n.d. | 0/12 | 100 / 10e5 |
| **Tumor reduktion :** | **-** | **-** | **>4.3 log** | |
| | | | | |

| Patient 3 | | | | |
|---|---|---|---|---|
| 24 | 6/6 | 6/6 | 2/6 | 4000/4x 10e6 |
| 24 | 6/6 | 6/6 | 1/6 | 1000/10e6 |
| 96 | 12/12 | 12/12 | 0/12 | 500/5 x 10e5 |
| 96 | 12/12 | 12/12 | 0/12 | 100/10e5 |
| **Tumor reduktion :** | **-** | **-** | **3 log** | |
| a) Anzahl der positiven Löcher (Tumorwachstum) von 6 bzw. 12 angesetzten Löchern nach 14 Tagen Kultivierung. | | | | |

MNCL = Mono-nucleated-cells

Das erfindungsgemäße Verfahren ist nicht nur zur Verringerung von kontaminierenden Tumorzellen in Stammzellpräparaten von Mammakarzinom-Patientinnen einsetzbar, obwohl es nachfolgend beispielhaft anhand von Mammakarzinompatientinnen beschrieben wird, sondern es kann beispielsweise auch zur Verringerung kontaminierender Tumorzellen in Stammzellpräparaten von Ovarialkrebspatienten oder von Patienten mit akuten oder chronischen Leukämien, Lymphomen, Hodenkarzinom oder anderen Chemotherapie sensitiven Karzinomen eingesetzt werden. Dem Fachmann sind die jeweiligen, bevorzugt zu verwendenden Antikörper bzw. Antikörperkombinationen bekannt oder sie können von ihm routinemäßig ohne erfinderisches Zutun ausgewählt und ermittelt werden.

Ziel der erfindungsgemäßen Behandlung ist somit die Heilung bzw. die Verlängerung eines krankheitsfreien Überlebens beispielsweise von Patientinnen mit Mammakarzinom, insbesondere von fortgeschrittenem Mammakarzinom, durch eine autologe Stammzelltransplantation. In einer bevorzugten Ausgestaltung der Erfindung sind die einzelnen Schritte, um dieses Ziel zu verfolgen, bei Diagenose eines Mammakarzinoms, beispielsweise wie folgt:
1. Nach Diagnose einer Metastasierung sollen - wenn immer möglich - Tumorzellen gewonnen werden. Nachweis der Antigene bzw. der Antigendichte von c-erb-B2 und ep-cam (epithelial cell adhesion molecule) auf nativen Tumorzellen mittels Durchflußzytometrie. Kryokonservierung von Tumorzellen.
2. Gewinnung autologer T-Lymphozyten aus dem peripheren Blut durch Leukapherese vor Beginn der Chemotherapie; Untersuchung einer möglichen Kontamination des Leukaphereseprodukts mit Tumorzellen durch Immunhistochemie und RT-PCR; Reinigung der T-Zell-Konzentrate von kontaminierenden Tumorzellen mit immunomagnetischen Beads.
3. 2 Zyklen einer Chemotherapie nach dem EC-Schema (Epirubicin 60 mg/m²+Cyclophosphamid 600 mg/m², gefolgt von G-CSF (5-10µg/kg/Tag)), Monitoring der Erholung der Hämatopoese und der Mobilisation von CD34+ Zellen nach den Zyklen.
4. Gewinnung hämatopoetischer Stammzellen aus dem Blut durch Leukapherese nach Mobilisation mit Epirubicin/Cyclophosphamid-Chemotherapie und G-CSF, wobei mindestens 4 x 10⁶ CD34+ Zellen/kg gewonnen werden sollen.
5. Zerstörung kontaminierender Tumorzellen im autologen Stammzellpräparat mit bispezifischen Antikörpern (anti-CD3 X anti-c-erbB-2 und anti-CD3 X anti-ep-cam, 500µg/Patient) durch 24-48h Inkubation bei Raumtemperatur und einer Zelldichte von 30.000-50.000 Zellen/µl. Kryokonservierung bei -180°C.
6. 2 Zyklen einer Chemotherapie nach dem ET-Schema (Epirubicin 60 mg/m² und Taxol 175 mg/m² gefolgt von G-CSF (5-10 mg/kg/Tag). Monitoring der Erholung der Hämatopoese und der Mobilisation der CD34+ Zellen. Evtl. Durchführung einer Leukapherese für ein Back-up-Präparat.
7. 3 Wochen nach der letzten Induktions-Chemotherapie myeloablative Hochdosis-Chemotherapie mit Thiotepa (600 mg/kg i.v.) und Melphalan (160 mg/kg i.v.).
8. Anschließende Reinfusion autologer Stammzellen.
9. 24h nach Reinfusion Gabe von 1 mg bsAk zur Restimulation der im Stammzelltransplantat aktivierten T-Zellen und Aufrechterhaltung der Anti-Tumorreaktion mit Vorkehrungen zur Prophylaxe und Behandlung von anaphylaktoiden Reaktionen. Haagen et al. konnten in in vitro-Versuchen zeigen, daß aufeinanderfolgende Gaben von bsAk innerhalb von 3 Tagen die Zerstörung von Tumorzellen signifikant erhöhen.
10. Reinfusion autologer T-Zellen nach der hämatopoetischen Rekonstitution (etwa an Tag 14-21) nach autologer Stammzelltransplantation. Verfolgung der T-Zell-Rekonstitution im peripheren Blut durch Durchflußzytometrie unter besonderer Brücksichtigung der CD45RA/CD45RO-Ratio der T-Helfer-Zellen.
11. Gabe von bispezifischen Antikörpern (200µg/1mg/2mg) in vivo in steigender Dosierung, an 3 aufeinanderfolgenden Tagen mit Vorkehrungen zur Prophylaxe und Behandlung von anaphylaktoiden Reaktionen.
12. Nachfolgeuntersuchungen: Beurteilung des Remissionsgrades, Anti-Antikörper-Reaktion gegen Immunglobulin von Maus und Ratte; Monitoring der hämatopoetischen Erholung und der Rekonstitution der T-Zell-Subpopulationen im peripheren Blut; regelmäßige Nachsorge zur Beurteilung der Dauer einer erreichten Remission. Versuche zum Nachweis von tumorspezifischen T-Zellen aus dem peripheren Blut.

Erfindungsgemäß werden somit bispezifische Antikörper zur Verminderung der Anzahl kontaminierender Tumorzellen, beispielsweise von Mammakarzinomzellen, durch deren Zerstörung in Stammzellpräparaten eingesetzt. Bei diesem Verfahren redigieren die Antikörper T-Zellen in die Nachbarschaft von Karzinomzellen und aktivieren die T-Zellen zur Sekretion von Zytokinen wie Tumor-Nekrose-Faktor α, die zur Lyse der Tumorzelle führen. Durch die Aktivierung von Makrophagen über den Fc-Rezeptor am Fc-Teil des bispezifischen Antikörpers wird dieser Zytokin-Effekt noch verstärkt; gleichzeitig könnten der T-Zelle wichtige kostimulatorische Signale von der FcγRI⁺-Zelle (Monozyt, Makrophage, Dendrit) übermittelt werden, die eine Anergisierung der T-Zelle verhindern.

Nach erfolgter Transplantation werden bispezifische Antikörper in steigender Dosierung nach der Reinfusion von autologen T-Zellen infundiert. In vivo kommt es zu einer Aktivierung von T-Zellen und Lyse residueller Mamma-Karzinomzellen. Hier könnten ebenfalls tumorspezifische T-Zellen expandiert werden (neben dem unter Punkt 1 erwähnten Stammzellpräparat), die bis dahin aufgrund einseitiger Stimulierung am Tumor über den T-Zellrezeptor ohne Kostimulus bzw. durch IL-10-Sekretion des Tumors anerg waren.

Nach heutigem Kenntnisstand kann die Immuntherapie ihre volle Wirkung gegen autologe Tumoren nur im Stadium einer geringen Resterkrankung entfalten; die Kombination der Immuntherapie mit Hochdosistherapie und autologer Stammzelltransplantation verspricht dafür die besten Aussichten. Aus diesem Grunde sollen die T-Zellen nach Abschluß der Chemo- bzw. Strahlentherapie infundiert werden.

Die Herstellung von bispezifischen Antikörpern gehört zum Stand der Technik. Beispielsweise können in einem neu entwikkelten Herstellungsverfahren (2) intakte bispezifische Antikörper in ausreichender Menge produziert werden. Die Kombination von 2 bispezifischen Antikörpern gegen 2 unterschiedliche tumorassoziierte Antigene (z. B. c-erb-B2, ep-cam, beispielsweise GA-733-2 = C215) auf den Mammakarzinomzellen minimiert die Gefahr, daß Tumorzellen, die nur ein Antigen exprimieren, unerkannt bleiben.

Das Risiko einer Reinfusion vitaler Tumorzellen mit dem T-Zellkonzentrat kann durch die Reinigung mit Antikörpern und immun-magnetischen Beads weitgehend ausgeschlossen werden. Geringe Mengen restlicher Tumorzellen sollen immunhistochemisch sowie mittels RT-PCR für das C215 Antigen bzw. CK19 nachgewiesen werden.

Wegen der möglichen Zytokinfreisetzung werden die bispezifischen Antikörper zuerst in niedriger Dosierung und unter strenger Kontrolle verabreicht. Berichten in der Literatur zufolge wurden vergleichbare bispezifische Antikörper in einer Menge bis zu 13 mg systemisch ohne wesentliche Nebenwirkungen verabreicht (1). Bei einer Antikörper-Menge von insgesamt 4 mg/Patient sind demnach kaum Nebenwirkungen zu erwarten.

Nachfolgend wird die Rolle bispezifischer Antikörper bei der Kombination von Chemo- und Immuntherapie zur Zerstörung residueller Mammakarzinomzellen im Transplantat und Patienten beschrieben.

Durch eine G-CSF Behandlung, die zur Mobilisierung von Stammzellen in die Peripherie eingesetzt wird, steigt ebenfalls die Anzahl von FcγRI-positiven Zellen (3). Dies beruht vor allem auf der durch G-CSF induzierten FcγRI-Expression auf neutrophilen Granulozyten. Der hochaffine Fcγ-Rezeptor I ist in der Lage, auch heterologe Ratte/Maus-bsAk der Isotypkombination Ratte-IgG2b und Maus-IgG2a zu binden (4). Diese Isotypkombination ist für die hier eingesetzten bsAk gewählt, um eine Bindung an die niedrig affineren, aber wesentlich stärker verbreiteten Fcγ-Rezeptoren vom Typ II und III zu verhindern (12), und damit die Gefahr einer unkontrollierten Zytokinfreisetzung zu minimieren. Eine Dosis von 13 mg eines bsAk mit der sich ähnlich verhaltenden Isotypkombination Ratte IgG2b und Maus IgG1 wurde bereits in einer Phase I-Studie an Patienten getestet. Die toxischen Nebenwirkungen waren gering (Grad I in der WHO-Klassifizierung) , so daß die projektierte Dosis von 4 mg/Patient gut vertragen werden sollte.

Die Bindung des bsAk an Fcγ-RI besitzt zwei wesentliche Vorteile im Hinblick auf eine optimale Anti-Tumorwirksamkeit:
1) FcγRI-positive Zellen besitzen die Fähigkeit, mittels ADCC Tumorzellen zu eliminieren (3), und können insofern synergistisch zur Anti-Tumorwirkung der durch den bsAk an die Tumorzelle herangeführten cytotoxischen T-Zellen beitragen (5).
2) Fcγ-RI-positive Zellen sind (wie z.B. Monozyten/Dendriten) in der Lage, wichtige kostimulatorische Signale, ähnlich wie bei der Antigen-Präsentation, der T-Zelle zu liefern und damit eine Anergisierung der T-Zelle zu verhindern. Wie in Abbildung 1 gezeigt, könnten weiterhin, als erwünschtes Nebenprodukt, aufgrund der durch intakten bsAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monozyt) geliefert werden. Insofern sollte der hier vorgestellte Ansatz neben der entscheidenden, direkten, T-Zellrezeptor-unabhängigen, durch bsAk vermittelten Tumorzerstörung (Abb.1A) ebenfalls tumorspezifische T-Zellen aktivieren und generieren (Abb. 1B), die nach Abbau der bsAk weiterhin im Patienten patrouillieren. D.h. mittels intakter bsAk könnte ähnlich wie bei gentherapeutischen Ansätzen (z.B. durch Einbau von kostimulatorischen Antigenen wie B-7 in die Tumorzelle) die Tumortoleranz im Patienten durchbrochen werden. Die in Beispiel 1 gezeigten Versuchsergebnisse im syngenen Tiermodell stützen diese Hypothese.

Günstig in diesem Zusammenhang ist weiterhin, daß die Expression von Fcγ-RI nach G-CSF-Behandlung auf den entsprechenden Zellen hochreguliert wird und bsAk mit Fc-Anteil eine wesentlich längere Zirkulationszeit als z.B. bsF(ab')2 oder bs(scFv) Antikörperfragmente besitzen, so daß wesentlich geringere Dosen intakter Ak-Moleküle für eine vergleichbare Anti-Tumorwirkung notwendig sind.

In dem hier vorgestellten Therapieansatz sollen residuelle Tumorzellen/Mikrometastasen zerstört werden. Im Gegensatz zur Behandlung solider Tumoren oder größerer Metastasen, bei denen die obenerwähnten Antikörperfragmente Vorteile besitzen könnten, da sie aufgrund ihrer geringeren Größe eine bessere Tumorpenetration ermöglichen, sind in der Situation der Mikrometastasierung intakte bsAk mit ihren bekannten Fc-Anteil-abhängigen Effektormechanismen vorzuziehen.

Weiterhin vorteilhaft ist die Tatsache, daß in dem hier vorgestellten Applikationsschema (bis zu zwei Tagen in vitro-Inkubation der autologen PBSZ mit 500 µg bsAk) keine die Therapie inhibierenden Anti-Antikörper zu erwarten sind. Da der Patient sich nach autologer Stammzelltransplantation in einem immunsupprimierten Zustand befindet, ist anzunehmen, daß gegen die 2 Wochen nach Transplantation in Kombination mit den autologen T-Zellen verabreichten bsAk noch keine Anti-Antikörper existieren.

Negative Auswirkungen der Immuntherapie mit bsAk auf das Wiederanwachsen der autologen Stammzellen sind aufgrund der gewählten Antikörperspezifitäten nicht zu erwarten.

Weiterhin können intakte bsAk durch Fusion von Ratte- und Maus-Hybridomen und anschließender Ein-Schritt-Reinigung über Protein A kostengünstig in klinikrelevanten Mengen produziert werden.

### Literatur:

1. Weiner & De Gast, Bispecific monoclonal antibody therapy of B-cell malignancy, Leukaemia and Lymphoma, 1995, 16:199
2. Lindhofer et al, Preferential species-restricted heavy-light chain pairing in rat-mouse quadromas: Implications for a single step purification of bispecific antibodies, J. Immunology 1995, 155:219
3. Valerius et al., Involvement of the high-affinity receptor for IgG (FcgRI; CD64) in enhanced tumor cell cytotoxicity of neutrophils during granulocyte colony-stimulating factor therapy, Blood, 1993, 82:931-939
4. Haagen et al., Interaction of human monocyte Fcg receptors with rat IgG2b, J.Immunology., 1995, 154: 1852-1860
5. Weiner et al.,The role of T cell activation in anti-CD3 x antitumor bispecific antibody therapy, J. Immunology, 1994, 152:2385
6. Kaneko et al. Combination of IL-2 stimulated lymphocytes and bispecific antibodies that efficiently lyse leukemic cells does not affect bone marrow CD34-positive stem cell function in vitro. Bone Marrow Transpl.1994, 14:213
7. Gale et al. Autotransplants in leukaemia. Lancet 1989, ii:315
8. Gribben et al. Immunologic purging of marrow assessed by PCR before autologous BMT for B cell lymphoma. New Engl. J. Med. 1991, 325:1525

## Patentansprüche

1. Verfahren zur Verminderung der Anzahl kontaminierender Tumorzellen in Stammzelltransplantaten ex vivo,
**dadurch gekennzeichnet,**
**daß** intakte bispezifische Antikörper, die sowohl an den T-Zellrezeptor-Komplex einer T-Zelle, an den Fc-Rezeptor von Fc-Rezeptor positiven Zellen als auch an tumorassoziierte Antigene auf einer Tumorzelle binden können, für einen ausreichend langen Zeitraum mit Stammzelltransplantaten, die kontaminierende Tumorzellen enthalten können, in Kontakt gebracht werden, um die Anzahl an kontaminierenden Tumorzellen im Stammzelltransplantat zumindest zu verringern.

2. Verfahren nach Anspruch 1,
**dadurch gekennzeichnet,**
**daß** Stammzelltransplantate aus Patientinnen mit einem Mammakarzinom oder Ovarialkarzinom oder aus Patienten mit Leukämien verwendet werden.

3. Verfahren nach Anspruch 1 oder Anspruch 2,
**dadurch gekennzeichnet,**
**daß** als bispezifische Antikörper anti-CD3 X anti-c-erB-2- und/oder anti-CD3 X anti-ep-cam- und/oder anti CD3 X anti Lewis Y-Antikörper verwendet werden.

4. Verfahren nach einem oder mehreren der vorhergehenden Ansprüche,
**dadurch gekennzeichnet,**
**daß** das Stammzelltransplantat mit den bispezifischen Antikörpern für einen Zeitraum von 4 - 72, insbesondere 24-48 Stunden inkubiert wird.

5. Verfahren nach einem oder mehreren der vorhergehenden Ansprüche,
**dadurch gekennzeichnet,**
**daß** die Inkubation bei einer Temperatur von 20 - 25°C, bevorzugt bei Raumtemperatur, erfolgt.

6. Verfahren nach einem oder mehreren der vorhergehenden Ansprüche,
**dadurch gekennzeichnet,**
**daß** die Zellen im Stammzelltransplantat in einer Dichte von 30.000 bis 75.000 Zellen/µl vorliegen.

7. Verwendung von intakten bispezifischen Antikörpern, die sowohl an den T-Zellrezeptor-Komplex einer T-Zelle, über ihren Fc-Teil an den Fc-Rezeptor von Fc-Rezeptor positiven Zellen als auch an tumorassoziierte Antigene auf einer Tumorzelle binden können, zur Verringerung der Anzahl kontaminierender Tumorzellen in Stammzelltransplantaten ex vivo.

8. Verwendung nach Anspruch 7,
**dadurch gekennzeichnet,**
**daß** die Stammzelltransplantate aus Patientinnen mit Mammakarzinom und/oder Ovarialkarzinom oder aus Patienten mit akuten oder chronischen Leukämien, Lymphomen, Hodenkarzinom oder anderen Chemotherapie sensitiven Karzinomen gewonnen werden.

9. Verwendung des in einem Verfahren der Ansprüche 1 - 6 erhaltenen Stammzelltransplantats zur Herstellung eines Arzneimittels zur Reinfusion in den Patienten.

## Claims

1. A method for the reduction of the number of contaminating tumour cells in stem cell transplants ex vivo,
**characterized in that**
intact bispecific antibodies able to bind to the T cell receptor complex of a T cell, to the Fc-receptor of a Fc-receptor positive cell as well as to tumour-associated antigens on a tumour cell are contacted with stem cell transplants which may contain contaminating tumour cells for a time sufficient to at least reduce the number of contaminating tumour cells in the stem cell transplant.

2. Method according to claim 1,
**characterized in that**
stem cell transplants from patients suffering from mamma carcinoma or ovarian carcinoma or from suffering from patients leukaemias are used.

3. Method according to claim 1 or claim 2,
**characterized in that**
anti-CD3 X anti-c-erbB-2 antibodies and/or anti-CD3 X anti-ep-cam antibodies and/or anti-CD3 X anti-Lewis Y antibodies are used as the bispecific antibodies.

4. Method according to one or more of the preceding claims,
**characterized in that**
said stem cell transplant is incubated with the bispecific antibodies for a period of 4 - 72 hours, in particular 24 - 48 hours.

5. Method according to one or more of the preceding claims,
**characterized in that**
said incubation is carried out at a temperature of 20 - 25° C, preferably at room temperature.

6. Method according to one or more of the preceding claims,
**characterized in that**
the cells in the stem cell transplant are present in a density of 30,000 to 75,000 cells per µl.

7. Use of intact bispecific antibodies able to bind to the T cell receptor complex of a T cell, via their Fc-part to the Fc-receptor of a Fc-receptor positive cell as well as to tumour-associated antigens on a tumour cell for the reduction of the number of contaminating tumour cells in stem cell transplants ex vivo.

8. Use according to claim 7,
**characterized in that**
said stem cell transplants are obtained from female patients with mamma carcinoma and/or ovarian carcinoma or from patients with acute or chronic leukaemias, lymphomas, testicular carcinoma or other carcinomas sensitive to chemotherapy.

9. Use of the stem cell transplant obtained in a method according to claims 1 to 6, for the preparation of a medicament for reinfusion into the patient.

## Revendications

1. Procédé pour la diminution du nombre de cellules tumorales contaminantes dans des greffons de cellules souches ex vivo, **caractérisé en ce que** des anticorps bispécifiques intacts, qui peuvent se fixer aussi bien au complexe de récepteur de lymphocyte T d'un lymphocyte T, au récepteur Fc de cellules récepteur Fc-positives qu'à des antigènes associés à des tumeurs sur une cellule tumorale, sont mis en contact, pendant une durée suffisante, avec des greffons de cellules souches qui peuvent contenir des cellules tumorales contaminantes, pour au moins réduire le nombre de cellules tumorales contaminantes dans le greffon de cellules souches.

2. Procédé selon la revendication 1, **caractérisé en ce qu'**on utilise des greffons de cellules souches provenant de patientes souffrant d'un carcinome mammaire ou d'un carcinome de l'ovaire, ou de patients à leucémies.

3. Procédé selon la revendication 1 ou 2, **caractérisé en ce qu'**on utilise comme anticorps bispécifiques les anticorps anti-CD3 X anti-c-erB-2 et/ou anti-CD3 X anti-ep-cam et/ou anti-CD3 X anti-Lewis Y.

4. Procédé selon une ou plusieurs des revendications précédentes, **caractérisé en ce que** le greffon de cellules souches est mis à incuber avec les anticorps bispécifiques pendant une durée de 4-72, en particulier 24-48 heures.

5. Procédé selon une ou plusieurs des revendications précédentes, **caractérisé en ce que** l'incubation est effectuée à une température de 20-25°C, de préférence à la température ambiante.

6. Procédé selon une ou plusieurs des revendications précédentes, **caractérisé en ce que** les cellules sont présentes dans le greffon de cellules souches à une densité de 30 000 à 75 000 cellules/µl.

7. Utilisation d'anticorps bispécifiques intacts, qui peuvent se fixer aussi bien au complexe de récepteur de lymphocytes T d'un lymphocyte T, par leur partie Fc au récepteur Fc de cellules récepteur Fc-positives, qu'à des antigènes associés à des tumeurs sur une cellule tumorale, pour réduire le nombre de cellules tumorales contaminantes dans des greffons de cellules souches ex vivo.

8. Utilisation selon la revendication 7, **caractérisée en ce que** les greffons de cellules souches sont obtenus à partir de patientes à carcinome mammaire et/ou carcinome de l'ovaire ou de patients à leucémies aiguës ou chroniques, lymphomes, carcinome du testicule ou autres carcinomes sensibles à la chimiothérapie.

9. Utilisation du greffon de cellules souches obtenu dans un procédé des revendications 1 à 6, pour la fabrication d'un médicament destiné à la reperfusion chez des patients.
